# EUROPÄISCHE PATENTANMELDUNG

(11) **EP 2 198 836 A1**
(43) Veröffentlichungstag der Anmeldung: **23.06.2010**
(21) Anmeldenummer: 09172315.5
(22) Anmeldetag: 06.10.2009
(51) Int. Cl.: A61K 8/25, A61K 8/44, A61K 8/73, A61K 8/86, A61Q 11/00

(54) **Remineralisierende Mund- und Zahnpflege- und -reinigungsmittel mit spezieller Wirkstoffkombination**

(30) Priorität: 16.12.2008 DE 102008062238
(71) Anmelder: Henkel AG & Co. KGaA, 40589 Düsseldorf (DE)
(72) Erfinder: Poth, Tilo, 69469, Weinheim (DE); Kliß, Rainer, 64354, Reinheim-Zeilhard (DE); Barth, Adolf Peter, 42781, Haan (DE)

(57) **Zusammenfassung**

Hochwirksame remineralisierende Mund- und Zahnpflege- und -reinigungsmittel, enthalten - bezogen auf sein Gewicht - 0,1 bis 5,0 Gew.-% Na-Carboxymethylcellulose mit einem Substitutionsgrad von 0,65 bis 0,85; 2,0 bis 25,0 Gew.-% Fällungskieselsäure(n); 0,1 bis 2 Gew.-% mindestens eines Tensids; 0,05 bis 5,0 Gew.-% mindestens eines Emulgators der Formel (I)

R-C(O)O-CH₂CH(OH)-CH₂(OCH₂CH₂)ₙOH (I),

in der R für einen langkettigen Alkyl- oder Alkenylrest mit 7 bis 21 Kohlenstoffatomen und n für Werte von 10 bis 100 steht, sowie 0,5 bis 5,0 Gew.-% mindestens eines Polyalkylenglycols der Formel (II)

H-(O-CH(R)-CH₂)m-OH (II),

in der R für -H oder -CH₃ steht undM so gewählt ist, daß das Polyalkylenglycol Molmassen zwischen 200 und 2000 Dalton aufweist.

## Beschreibung

Die Erfindung betrifft Zubereitungen zur Mund- und Zahnpflege- und -reinigung, die aufgrund von speziellen Inhaltsstoffen remineralisierend wirken, eine hohe Reinigungsleistung besitzen, ohne die Zahnoberfläche zu zerkratzen oder zu sehr abrasiv zu wirken und darüber hinaus die Entstehung von Mundgeruch verhindern und Mundgeruch verringern.

Zahnreinigungsmittel sind in verschiedenen Formen auf dem Markt und dienen in erster Linie der Reinigung der Zahnoberfläche und der Vorbeugung von Zahn- und Zahnfleischerkrankungen. Sie enthalten üblicherweise eine Kombination aus Poliermitteln, Feuchthaltemitteln, Tensiden, Bindemitteln, Aromastoffen und fluoridhaltigen sowie antimikrobiellen Wirkstoffen. Neben Zahnpulvern, die wegen ihrer erhöhten Abrasivität eine untergeordnete Rolle spielen, werden Zahnreinigungsmittel vor allem in Pasten-, Creme- und transluzenter oder transparenter Gelform angeboten. In den letzten Jahren haben auch Liquid- oder Flüssigzahncremes und Mundwässer zunehmend an Bedeutung gewonnen.

Neben der Reinigung der Zähne erwartet der Verbraucher von den gattungsgemäßen Produkten auch eine Pflege der Zähne und der Mundhöhle. So sind insbesondere ein "sauberes" Gefühl, d.h. eine glatte und glänzende Zahnoberfläche sowie ein frisches Gefühl im Mund wesentliche Aspekte für den Kaufanreiz von Zubereitungen zur Mund- und Zahnpflege- und -reinigung. Ein erfolgreiches Mittel der gattungsgemäßen Art sollte daher die Zähne gründlich reinigen, ohne den Zahn oder die Zahnoberfläche zu schädigen und gleichzeitig Mundgeruch verringern und/oder verhindern.

Eine Aufgabe, die Zahnreinigungsmittel neben der bloßen Reinigung der Zahnoberfläche erfüllen sollten, ist die Förderung der Remineralisierung des Zahnschmelzes und die Festigung des Zahnschmelzes und damit die Kariesprophylaxe. Weiterhin soll durch die Reminealisierung und den Verschluss von Dentalläsionen auch die Empfindlichkeit der Zähne gegen taktile, sensorische oder chemische Reize (Berührung, Kälte/Wärme, saure oder stark zuckerhaltige Speisen) verringert werden.

Es bestand daher nach wie vor die Aufgabe, die vorstehend genannten Probleme zu lösen, ohne hohe Mengen gegebenenfalls teurer Inhaltstoffe in die Formulierungen einarbeiten zu müssen. Darüber hinaus bestand die Aufgabe der vorliegenden Erfindung darin, Mund- und Zahnpflege-und -reinigungsmittel bereitzustellen, welche die Zähne remineralisren und gegen äußere Einflüsse unempfindlicher machen.

Es wurde nun gefunden, daß eine spezielle Kombination von Inhaltsstoffen zu deutlich verbesserter Remineralisation beiträgt.

Gegenstand der vorliegenden Erfindung ist in einer ersten Ausführungsform ein Mund- und Zahnpflege- und -reinigungsmittel, enthaltend - bezogen auf sein Gewicht -
a) 0,1 bis 5,0 Gew.-% Na-Carboxymethylcellulose mit einem Substitutionsgrad von 0,65 bis 0,85;
b) 2,0 bis 25,0 Gew.-% Fällungskieselsäure(n);
c) 0,1 bis 2 Gew.-% mindestens eines Tensids;
d) 0,05 bis 5,0 Gew.-% mindestens eines Emulgators der Formel (I)

   R-C(O)O-CH₂CH(OH)-CH₂(OCH₂CH₂)ₙOH (I),

   in der
   - R für einen langkettigen Alkyl- oder Alkenylrest mit 7 bis 21 Kohlenstoffatomen und
   - n für Werte von 10 bis 100 steht,
e) 0,5 bis 5,0 Gew.-% mindestens eines Polyalkylenglycols der Formel (II)

   H-(O-CH(R)-CH₂)m-OH (II),

   in der
   - R für-H oder -CH₃ steht,
   - M so gewählt ist, daß das Polyalkylenglycol Molmassen zwischen 200 und 2000 Dalton aufweist.

Mund- und Zahnpflegemittel sowie Mund- und Zahnreinigungsmittel im Sinne der Erfindung sind Mund- und Zahnpulver, Mund- und Zahnpasten, flüssige Mund- und Zahncremes sowie Mund- und Zahngele. Bevorzugt geeignet sind Zahnpasten und flüssige Zahnreinigungsmittel. Hierzu können die Mund- und Zahnpflege- und reinigungsmittel z.B. in Form von Zahnpasten, flüssigen Zahncremes, Zahnpulvern, Mundwässern oder gegebenenfalls auch als Kaumasse, z. B. als Kaugummi, vorliegen. Bevorzugt liegen sie jedoch als mehr oder weniger fließfähige oder plastische Zahnpasten vor, wie sie zur Reinigung der Zähne unter Einsatz einer Zahnbürste verwendet werden.

Die erfindungsgemäßen Mund- und Zahnpflege- und -reinigungsmittel enthalten eine besonders remineralisierend wirkende Kombination aus fünf Inhaltsstoffen. Diese werden nachstehend beschrieben:

Als ersten wesentlichen Inhaltsstoff enthalten die erfindungsgemäßen Mund- und Zahnpflege-und -reinigungsmittel Natrium-Carboxymethylcellulose. Diese wird - bezogen auf das Gewicht des anwendungsfertigen Mund- und Zahnpflege- und -reinigungsmittels - in Mengen von 0,1 bis 5,0 Gew.-% eingesetzt und weist Substitutionsgrade von 0,65 bis 0,85 auf.

Na-Carboxymethylcellulose ist das Natriumsalz des Glycolsäureethers der Cellulose:

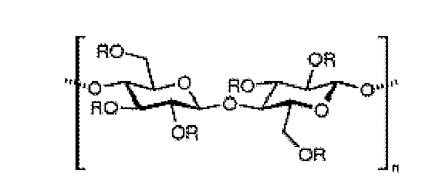

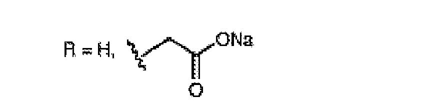

Na-Carboxymethylcellulose wird technisch hergestellt durch Umsetzung von Alkalicellulose mit Monochloressigsäure bzw. deren Natriumsalz. Der Substituionsgrad gibt an, wie viele der Reste R in der o.g. Formel für Wasserstoffatome bzw. -CH₂-COONa-Gruppierungen stehen. Ein Substitutionsgrad von 1 bedeutet dabei, daß einer der sechs Reste R pro wiederkehrender Formeleinheit für eine -CH₂-COONa-Gruppierung steht, während die restlichen fünf Reste -H sind.

Es ist erfindungsgemäß bevorzugt, die Na-CMC in engeren Mengenbereichen einzusetzen. Besonders bevorzugt sind - bezogen auf das Gewicht des anwendungsfertigen Mund- und Zahnpflege- und -reinigungsmittels - Mengen von 0,25 bis 4,0 Gew.-%, vorzugsweise von 0,5 bis 3,0 Gew.-%, besonders bevorzugt von 0,75 bis 2,5 und insbesondere von 1,0 bis 1,75 Gew.-%.

Unabhängig von der Menge der eingesetzten Na-CMC sind Carboxymethycellulosen bevorzugt, die einen Substitutionsgrad von 0,75 bis 0,85 aufweisen.

Zusammenfassend sind erfindungsgemäß bevorzugte Mund- und Zahnpflege- und - reinigungsmittel **dadurch gekennzeichnet, daß** sie - bezogen auf ihr Gewicht - 0,25 bis 4,0 Gew.-%, vorzugsweise 0,5 bis 3,0 Gew.-%, besonders bevorzugt 0,75 bis 2,5 und insbesondere 1,0 bis 1,75 Gew.-% Na-Carboxymethylcellulose mit einem Substitutionsgrad von 0,75 bis 0,85 enthalten.

Als zweiten wesentlichen Inhaltsstoff enthalten die erfindungsgemäßen Mund- und Zahnpflege-und -reinigungsmittel mindestens eine Fällungskieselsäure.

Fällungskieselsäuren werden durch Ausfällung von Kieselsäure aus verdünnten Alkalisilikat-Lösungen durch Zugabe von starken Säuren unter Bedingungen erhalten, bei welchen die Aggregation zum Sol und Gel nicht eintreten kann. Bevorzugt geeignet ist eine Fällungskieselsäure mit einer BET-Oberfläche von 15 - 110 m²/g, einer Partikelgröße von 0,5 - 20 µm, wobei wenigstens 80 Gew.-% der Primärpartikel unter 5 µm liegen sollen, und einer Viskosität in 30 %iger Glycerin-Wasser-(1 : 1)-Dispersion von 30 - 60 Pa.s (20°C) in einer Menge von 10 - 20 Gew.-% der Zahnpaste. Bevorzugt geeignete Fällungskieselsäuren dieser Art weisen außerdem gerundete Ecken und Kanten auf und sind unter der Handelsbezeichnung Sident^{®}12 DS (DEGUSSA) erhältlich.

Andere Fällungskieselsäuren dieser Art sind Sident^{®} 8 (DEGUSSA) und Sorbosil^{®} AC 39 (Crosfield Chemicals). Diese Kieselsäuren zeichnen sich durch eine geringere Verdickungswirkung und eine etwas höhere mittlere Teilchengröße von 8 --14 µm bei einer spezifischen Oberfläche von 40 - 75 m²/g (nach BET) aus und eignen sich besonders gut für flüssige Zahncremes. Diese sollten eine Viskosität (25°C, Scherrate D = 10 s⁻¹) von 10 - 100 Pa.s aufweisen.

Andere besonders bevorzugt geeignete Fällungskieselsäuren dieser Art sind Sident^{®} 22 S (DEGUSSA). Diese Kieselsäuren zeichnen sich durch eine geringere Verdickungswirkung und eine etwas höhere mittlere Teilchengröße von 7 µm bei einer spezifischen Oberfläche von ca. 190 m²/g (nach BET) aus.

Zahnpasten, die eine deutlich höhere Viskosität von mehr als 100 Pa.s (25° C, D = 10 s⁻¹) aufweisen, benötigen hingegen einen genügend hohen Anteil an Kieselsäuren mit einer Teilchengröße von weniger als 5 µm, bevorzugt wenigstens 3 Gew.-% einer Kieselsäure mit einer Partikelgröße von 1 - 3 µm. Solchen Zahnpasten setzt man daher bevorzugt neben den genannten Fällungskieselsäuren noch feinteiligere, so genannte Verdickungskieselsäuren mit einer BET-Oberfläche von 150 -250 m²/g zu, z.B. die Handelsprodukte Sipernat 22 LS oder Sipernat^{®} 320 DS.

Erfindungsgemäß besonders bevorzugte Mund- und Zahnpflege- und -reinigungsmittel enthalten - bezogen auf ihr Gewicht - 2,5 bis 22,5, vorzugsweise 3,5 bis 20, besonders bevorzugt 5 bis 17,5 und insbesondere 6,0 bis 15,0 Gew.-% Fällungskieselsäure(n).

Als dritten wesentlichen Inhaltsstoff enthalten die erfindungsgemäßen Mund- und Zahnpflege-und -reinigungsmittel mindestens ein Tensid. Unter den Tensiden sind anionische und/oder amphotere Tenside besonders bevorzugt. Ganz besonders bevorzugt sind Fettalkoholsulfate und/oder Betaintenside.

Besonders bevorzugte erfindungsgemäße Mund- und Zahnpflege- und -reinigungsmittel sind **dadurch gekennzeichnet, daß** sie - bezogen auf ihr Gewicht - 0,25 bis 1,9 Gew.-%, vorzugsweise 0,5 bis 1,8 Gew.-%, besonders bevorzugt 0,75 bis 1,7 und insbesondere 1,0 bis 1,5 Gew.-% mindestens eines Betains der Formel (III) 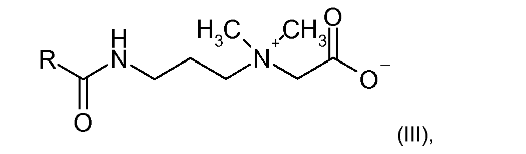 enthalten, in der R für einen geradkettigen oder verzweigten, gesättigten oder ein- bzw. mehrfach ungesättigten Alkyl- oder Alkenlyrest mit 8 bis 24 Kohlenstoffatomen steht.

Diese Tenside werden nach der INCI-Nomenklatur als Amidopropylbetaine bezeichnet, wobei die Vertreter, die sich von Kokosfettsäuren ableiten, bevorzugt sind und als Cocoamidopropylbetaine bezeichnet werden. Besonders bevorzugt werden erfindungsgemäß Tenside der Formel (III) eingesetzt, die ein Gemisch der folgenden Vertreter sind:
H₃C-(CH₂)₇-C(O)-NH-(CH₂)₃N⁺(CH₃)₂CH₂COO⁻
H₃C-(CH₂)₉-C(O)-NH-(CH₂)₃N⁺(CH₃)₂CH₂COO⁻
H₃C-(CH₂)₁₁-C(O)-NH-(CH₂)₃N⁺(CH₃)₂CH₂COO⁻
H₃C-(CH₂)₁₃-C(O)-NH-(CH₂)₃N⁺(CH₃)₂CH₂COO⁻
H₃C-(CH₂)₁₅-C(O)-NH-(CH₂)₃N⁺(CH₃)₂CH₂COO⁻
H₃C-(CH₂)₇-CH=CH-(CH₂)₇-C(O)-NH-(CH₂)₃N⁺(CH₃)₂CH₂COO⁻

Zusätzlich zu dem bzw. den Amidopropylbetainen oder an ihrer Stelle können die erfindungsgemäßen Mittel auch Amphoacetate enthalten. Hier sind erfindungsgemäße Mund- und Zahnpflege- und -reinigungsmittel bevorzugt, die - bezogen auf ihr Gewicht - 0,25 bis 1,9 Gew.-%, vorzugsweise 0,5 bis 1,8 Gew.-%, besonders bevorzugt 0,75 bis 1,7 und insbesondere 1,0 bis 1,5 Gew.-% mindestens eines Betains der Formel (IV) 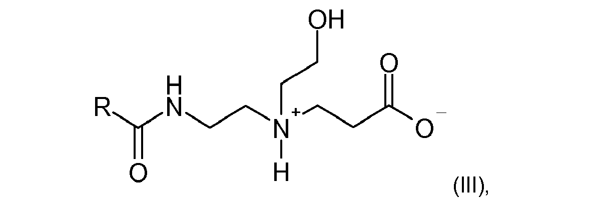 enthalten, in der R für einen geradkettigen oder verzweigten, gesättigten oder ein- bzw. mehrfach ungesättigten Alkyl- oder Alkenlyrest mit 8 bis 24 Kohlenstoffatomen steht.

Diese Tenside werden nach der INCI-Nomenklatur als Amphoacetate bezeichnet, wobei die Vertreter, die sich von Kokosfettsäuren ableiten bevorzugt sind und als Cocoamphoactetate bezeichnet werden.

Aus herstellungstechnischen Gründen enthalten Tenside dieses Typs immer auch Betaine der Formel (IVa)

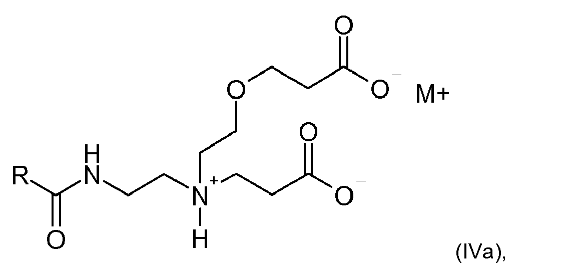

in der R für einen geradkettigen oder verzweigten, gesättigten oder ein- bzw. mehrfach ungesättigten Alkyl- oder Alkenlyrest mit 8 bis 24 Kohlenstoffatomen und M für ein Kation steht.

Diese Tenside werden nach der INCI-Nomenklatur als Amphodiacetate bezeichnet, wobei die Vertreter, die sich von Kokosfettsäuren ableiten, bevorzugt sind und als Cocoamphodiactetate bezeichnet werden.

Besonders bevorzugt werden erfindungsgemäß Tenside der Formel (IV) eingesetzt, die ein Gemisch der folgenden Vertreter sind:
H₃C-(CH₂)₇-C(O)-NH-(CH₂)₂NH⁺(CH₂CH₂OH)CH₂CH₂COO⁻
H₃C-(CH₂)₉-C(O)-NH-(CH₂)₂NH⁺(CH₂CH₂OH)CH₂CH₂COO⁻
H₃C-(CH₂)₁₁-C(O)-NH-(CH₂)₂NH⁺(CH₂CH₂OH)CH₂CH₂COO⁻
H₃C-(CH₂)₁₃-C(O)-NH-(CH₂)₂NH⁺(CH₂CH₂OH)CH₂CH₂COO⁻
H₃C-(CH₂)₁₅-C(O)-NH-(CH₂)₂NH⁺(CH₂CH₂OH)CH₂CH₂COO⁻
H₃C-(CH₂)₇-CH=CH-(CH₂)₇-C(O)-NH-(CH₂)₂NH⁺(CH₂CH₂OH)CH₂CH₂COO⁻

Zusammenfassend sind erfindungsgemäße Mund- und Zahnpflege- und -reinigungsmittel bevorzugt, bei denen der Rest R in den Formeln (III) und (IV) ausgewählt ist aus
- H₃C-(CH₂)₇-
- H₃C-(CH₂)₉-
- H₃C-(CH₂)₁₁-
- H₃C-(CH₂)₁₃-
- H₃C-(CH₂)₁₅-
- H₃C-(CH₂)₇-CH=CH-(CH₂)₇-
oder Mischungen aus diesen.

Zusätzlich zu dem bzw. den Betaintensiden oder an ihrer Stelle können die erfindungsgemäßen Mittel auch anionische Tenside enthalten. Hier sind die Fettalkoholsulfate besonders bevorzugt. Besonders bevorzugte erfindungsgemäße Mund- und Zahnpflege- und -reinigungsmittel sind **dadurch gekennzeichnet, daß** sie - bezogen auf ihr Gewicht - 0,15 bis 1,9 Gew.-%, vorzugsweise 0,5 bis 1,8 Gew.-%, besonders bevorzugt 0,75 bis 1,7 und insbesondere 1,0 bis 1,5 Gew.-% mindestens eines anionischen Tensids aus der Gruppe der Fettalkoholsulfate enthalten.

Als vierten wesentlichen Inhaltsstoff enthalten die erfindungsgemäßen Mund- und Zahnpflege-und -reinigungsmittel mindestens 0,05 bis 5,0 Gew.-% mindestens eines Emulgators der Formel (I)

R-C(O)O-CH₂CH(OH)-CH₂(OCH₂CH₂)ₙOH (I),

in der R für einen langkettigen Alkyl- oder Alkenylrest mit 7 bis 21 Kohlenstoffatomen und n für Werte von 10 bis 100 steht.

Es ist erfindungsgemäß bevorzugt, diese Emulgatoren in engeren Mengenbereichen einzusetzen. Besonders bevorzugt sind - bezogen auf das Gewicht des anwendungsfertigen Mund- und Zahnpflege- und -reinigungsmittels - Mengen von 0,07 bis 4,0 Gew.-%, vorzugsweise 0,1 bis 3,0 Gew.-%, besonders bevorzugt 0,15 bis 2,0 und insbesondere 0,2 bis 1,0 Gew.-%.

Unabhängig von der Menge der eingesetzten Emulgatoren der Formel (I) sind solche Emulgatoren bevorzugt, bei denen in Formel (I) R für -(CH2)ₖCH3 mit k 8, 10, 12, 14, 16 oder 18 und n für die Werte 20, 21, 22, 23, 24, 25, 26, 27, 28, 29, 30, 31, 32, 33, 34, 35, 36, 37, 38, 39 oder 40 steht.

Zusammenfassend sind erfindungsgemäße Mund- und Zahnpflege- und -reinigungsmittel bevorzugt, die - bezogen auf ihr Gewicht - 0,07 bis 4,0 Gew.-%, vorzugsweise 0,1 bis 3,0 Gew.-%, besonders bevorzugt 0,15 bis 2,0 und insbesondere 0,2 bis 1,0 Gew.-% mindestens eines Emulgators der Formel (I) enthalten

R-C(O)O-CH₂CH(OH)-CH₂(OCH₂CH₂)ₙOH (I),

in der
- R für -(CH₂)ₖCH₃ mit k 8, 10, 12, 14, 16 oder 18 und
- n für die Werte 20, 21, 22, 23, 24, 25, 26, 27, 28, 29, 30, 31, 32, 33, 34, 35, 36, 37, 38, 39 oder 40 steht.

Ganz besonders bevorzugt ist der Einsatz mindestens eines Emulgators aus der nachstehenden Liste:
H₃C-(CH₂)₈-C(O)O-CH₂CH(OH)-CH₂(OCH₂CH₂)₂₀OH
H₃C-(CH₂)₈-C(O)O-CH₂CH(OH)-CH₂(OCH₂CH₂)₂₁OH
H₃C-(CH₂)₈-C(O)O-CH₂CH(OH)-CH₂(OCH₂CH₂)₂₂OH
H₃C-(CH₂)₈-C(O)O-CH₂CH(OH)-CH₂(OCH₂CH₂)₂₃OH
H₃C-(CH₂)₈-C(O)O-CH₂CH(OH)-CH₂(OCH₂CH₂)₂₄0H
H₃C-(CH₂)₈-C(O)O-CH₂CH(OH)-CH₂(OCH₂CH₂)₂₅OH
H₃C-(CH₂)₈-C(O)O-CH₂CH(OH)-CH₂(OCH₂CH₂)₂₆OH
H₃C-(CH₂)₈-C(O)O-CH₂CH(OH)-CH₂(OCH₂CH₂)₂₇OH
H₃C-(CH₂)₈-C(O)O-CH₂CH(OH)-CH₂(OCH₂CH₂)₂₈OH
H₃C-(CH₂)₈-C(O)O-CH₂CH(OH)-CH₂(OCH₂CH₂)₂₉OH
H₃C-(CH₂)₈-C(O)O-CH₂CH(OH)-CH₂(OCH₂CH₂)₃₀OH
H₃C-(CH₂)₈-C(O)O-CH₂CH(OH)-CH₂(OCH₂CH₂)₃₁OH
H₃C-(CH₂)₈-C(O)O-CH₂CH(OH)-CH₂(OCH₂CH₂)₃₂OH
H₃C-(CH₂)₈-C(O)O-CH₂CH(OH)-CH₂(OCH₂CH₂)₃₃OH
H₃C-(CH₂)₈-C(O)O-CH₂CH(OH)-CH₂(OCH₂CH₂)₃₄OH
H₃C-(CH₂)₈-C(O)O-CH₂CH(OH)-CH₂(OCH₂CH₂)₃₅OH
H₃C-(CH₂)₈-C(O)O-CH₂CH(OH)-CH₂(OCH₂CH₂)₃₆OH
H₃C-(CH₂)₈-C(O)O-CH₂CH(OH)-CH₂(OCH₂CH₂)₃₇OH
H₃C-(CH₂)₈-C(O)O-CH₂CH(OH)-CH₂(OCH₂CH₂)₃₈OH
H₃C-(CH₂)₈-C(O)O-CH₂CH(OH)-CH₂(OCH₂CH₂)₃₉OH
H₃C-(CH₂)₈-C(O)O-CH₂CH(OH)-CH₂(OCH₂CH₂)₄₀OH
H₃C-(CH₂)₁₀-C(O)O-CH₂CH(OH)-CH₂(OCH₂CH₂)₂₀OH
H₃C-(CH₂)₁₀-C(O)O-CH₂CH(OH)-CH₂(OCH₂CH₂)₂₁OH
H₃C-(CH₂)₁₀-C(O)O-CH₂CH(OH)-CH₂(OCH₂CH₂)₂₂OH
H₃C-(CH₂)₁₀-C(O)O-CH₂CH(OH)-CH₂(OCH₂CH₂)₂₃OH
H₃C-(CH₂)₁₀-C(O)O-CH₂CH(OH)-CH₂(OCH₂CH₂)₂₄OH
H₃C-(CH₂)₁₀-C(O)O-CH₂CH(OH)-CH₂(OCH₂CH₂)₂₀OH
H₃C-(CH₂)₁₀-C(O)O-CH₂CH(OH)-CH₂(OCH₂CH₂)₂₆OH
H₃C-(CH₂)₁₀-C(O)O-CH₂CH(OH)-CH₂(OCH₂CH₂)₂₇OH
H₃C-(CH₂)₁₀-C(O)O-CH₂CH(OH)-CH₂(OCH₂CH₂)₂₈OH
H₃C-(CH₂)₁₀-C(O)O-CH₂CH(OH)-CH₂(OCH₂CH₂)₂₉OH
H₃C-(CH₂)₁₀-C(O)O-CH₂CH(OH)-CH₂(OCH₂CH₂)₃₀OH
H₃C-(CH₂)₁₀-C(O)O-CH₂CH(OH)-CH₂(OCH₂CH₂)₃₁OH
H₃C-(CH₂)₁₀-C(O)O-CH₂CH(OH)-CH₂(OCH₂CH₂)₃₂OH
H₃C-(CH₂)₁₀-C(O)O-CH₂CH(OH)-CH₂(OCH₂CH₂)₃₂OH
H₃C-(CH₂)₁₀-C(O)O-CH₂CH(OH)-CH₂(OCH₂CH₂)₃₄OH
H₃C-(CH₂)₁₀-C(O)O-CH₂CH(OH)-CH₂(OCH₂CH₂)₃₅OH
H₃C-(CH₂)₁₀-C(O)O-CH₂CH(OH)-CH₂(OCH₂CH₂)₃₆OH
H₃C-(CH₂)₁₀-C(O)O-CH₂CH(OH)-CH₂(OCH₂CH₂)₃₇OH
H₃C-(CH₂)₁₀-C(O)O-CH₂CH(OH)-CH₂(OCH₂CH₂)₃₈OH
H₃C-(CH₂)₁₀-C(O)O-CH₂CH(OH)-CH₂(OCH₂CH₂)₃₉OH
H₃C-(CH₂)₁₀-C(O)O-CH₂CH(OH)-CH₂(OCH₂CH₂)₄₀OH
H₃C-(CH₂)₁₂-C(O)O-CH₂CH(OH)-CH₂(OCH₂CH₂)₂₀OH
H₃C-(CH₂)₁₂-C(O)O-CH₂CH(OH)-CH₂(OCH₂CH₂)₂₁OH
H₃C-(CH₂)₁₂-C(O)O-CH₂CH(OH)-CH₂(OCH₂CH₂)_{22O}H
H₃C-(CH₂)₁₂-C(O)O-CH₂CH(OH)-CH₂(OCH₂CH₂)₂₃OH
H₃C-(CH₂)₁₂-C(O)O-CH₂CH(OH)-CH₂(OCH₂CH₂)₂₄OH
H₃C-(CH₂)₁₂-C(O)O-CH₂CH(OH)-CH₂(OCH₂CH₂)₂₅OH
H₃C-(CH₂)₁₂-C(O)O-CH₂CH(OH)-CH₂(OCH₂CH₂)₂₆OH
H₃C-(CH₂)₁₂-C(O)O-CH₂CH(OH)-CH₂(OCH₂CH₂)₂₇OH
H₃C-(CH₂)₁₂-C(O)O-CH₂CH(OH)-CH₂(OCH₂CH₂)₂₈OH
H₃C-(CH₂)₁₂-C(O)O-CH₂CH(OH)-CH₂(OCH₂CH₂)₂₉OH
H₃C-(CH₂)₁₂-C(O)O-CH₂CH(OH)-CH₂(OCH₂CH₂)₃₀OH
H₃C-(CH₂)₁₂-C(O)O-CH₂CH(OH)-CH₂(OCH₂CH₂)₃₁OH
H₃C-(CH₂)₁₂-C(O)O-CH₂CH(OH)-CH₂(OCH₂CH₂)₃₂OH
H₃C-(CH₂)₁₂-C(O)O-CH₂CH(OH)-CH₂(OCH₂CH₂)₃₃OH
H₃C-(CH₂)₁₂C(O)O-CH₂CH(OH)-CH₂(OCH₂CH₂)₃₄OH
H₃C-(CH₂)₁₂-C(O)O-CH₂CH(OH)-CH₂(OCH₂CH₂)₃₅OH
H₃C-(CH₂)₁₂-C(O)O-CH₂CH(OH)-CH₂(OCH₂CH₂)₃₆OH
H₃C-(CH₂)₁₂-C(O)O-CH₂CH(OH)-CH₂(OCH₂CH₂)₃₇OH
H₃C-(CH₂)₁₂-C(O)O-CH₂CH(OH)-CH₂(OCH₂CH₂)₃₈OH
H₃C-(CH₂)₁₂-C(O)O-CH₂CH(OH)-CH₂(OCH₂CH₂)₃₉OH
H₃C-(CH₂)₁₂-C(O)O-CH₂CH(OH)-CH₂(OCH₂CH₂)₄₀OH
H₃C-(CH₂)₁₄-C(O)O-CH₂CH(OH)-CH₂(OCH₂CH₂)₂₀OH
H₃C-(CH₂)₁₄-C(O)O-CH₂CH(OH)-CH₂(OCH₂CH₂)₂₁OH
H₃C-(CH₂)₁₄-C(O)O-CH₂CH(OH)-CH₂(OCH₂CH₂)₂₂OH
H₃C-(CH₂)₁₄-C(O)O-CH₂CH(OH)-CH₂(OCH₂CH₂)₂₃OH
H₃C-(CH₂)₁₄-C(O)O-CH₂CH(OH)-CH₂(OCH₂CH₂)₂₄OH
H₃C-(CH₂)₁₄-C(O)O-CH₂CH(OH)-CH₂(OCH₂CH₂)₂₅OH
H₃C-(CH₂)₁₄-C(O)O-CH₂CH(OH)-CH₂(OCH₂CH₂)₂₆OH
H₃C-(CH₂)₁₄-C(O)O-CH₂CH(OH)-CH₂(OCH₂CH₂)₂₇OH
H₃C-(CH₂)₁₄-C(O)O-CH₂CH(OH)-CH₂(OCH₂CH₂)₂₈OH
H₃C-(CH₂)₁₄-C(O)O-CH₂CH(OH)-CH₂(OCH₂CH₂)₂₉OH
H₃C-(CH₂)₁₄-C(O)O-CH₂CH(OH)-CH₂(OCH₂CH₂)₃₀OH
H₃C-(CH₂)₁₄-C(O)O-CH₂CH(OH)-CH₂(OCH₂CH₂)₃₁OH
H₃C-(CH₂)₁₄-C(O)O-CH₂CH(OH)-CH₂(OCH₂CH₂)₃₂OH
H₃C-(CH₂)₁₄-C(O)O-CH₂CH(OH)-CH₂(OCH₂CH₂)₃₃OH
H₃C-(CH₂)₁₄-C(O)O-CH₂CH(OH)-CH₂(OCH₂CH₂)₃₄OH
H₃C-(CH₂)₁₄-C(O)O-CH₂CH(OH)-CH₂(OCH₂CH₂)₃₅OH
H₃C-(CH₂)₁₄-C(O)O-CH₂CH(OH)-CH₂(OCH₂CH₂)₃₆OH
H₃C-(CH₂)₁₄-C(O)O-CH₂CH(OH)-CH₂(OCH₂CH₂)₃₇OH
H₃C-(CH₂)₁₄-C(O)O-CH₂CH(OH)-CH₂(OCH₂CH₂)₃₈OH
H₃C-(CH₂)₁₄-C(O)O-CH₂CH(OH)-CH₂(OCH₂CH₂)₃₉OH
H₃C-(CH₂)₁₄-C(O)O-CH₂CH(OH)-CH₂(OCH₂CH₂)₄₀OH
H₃C-(CH₂)₁₆-C(O)O-CH₂CH(OH)-CH₂(OCH₂CH₂)₂₀OH
H₃C-(CH₂)₁₆-C(O)O-CH₂CH(OH)-CH₂(OCH₂CH₂)₂₁OH
H₃C-(CH₂)₁₆-C(O)O-CH₂CH(OH)-CH₂(OCH₂CH₂)₂₂OH
H₃C-(CH₂)₁₆-C(O)O-CH₂CH(OH)-CH₂(OCH₂CH₂)₂₃OH
H₃C-(CH₂)₁₆-C(O)O-CH₂CH(OH)-CH₂(OCH₂CH₂)₂₄OH
H₃C-(CH₂)₁₆-C(O)O-CH₂CH(OH)-CH₂(OCH₂CH₂)₂₅OH
H₃C-(CH₂)₁₆-C(O)O-CH₂CH(OH)-CH₂(OCH₂CH₂)₂₆OH
H₃C-(CH₂)₁₆-C(O)O-CH₂CH(OH)-CH₂(OCH₂CH₂)₂₇OH
H₃C-(CH₂)₁₆-C(O)O-CH₂CH(OH)-CH₂(OCH₂CH₂)₂₈OH
H₃C-(CH₂)₁₆-C(O)O-CH₂CH(OH)-CH₂(OCH₂CH₂)₂₉OH
H₃C-(CH₂)₁₆-C(O)O-CH₂CH(OH)-CH₂(OCH₂CH₂)₃₀OH
H₃C-(CH₂)₁₆-C(O)O-CH₂CH(OH)-CH₂(OCH₂CH₂)₃₁OH
H₃C-(CH₂)₁₆-C(O)O-CH₂CH(OH)-CH₂(OCH₂CH₂)₃₂OH
H₃C-(CH₂)₁₆-C(O)O-CH₂CH(OH)-CH₂(OCH₂CH₂)₃₃OH
H₃C-(CH₂)₁₆-C(O)O-CH₂CH(OH)-CH₂(OCH₂CH₂)₃₄OH
H₃C-(CH₂)₁₆-C(O)O-CH₂CH(OH)-CH₂(OCH₂CH₂)₃₅OH
H₃C-(CH₂)₁₆-C(O)O-CH₂CH(OH)-CH₂(OCH₂CH₂)₃₆OH
H₃C-(CH₂)₁₆-C(O)O-CH₂CH(OH)-CH₂(OCH₂CH₂)₃₇OH
H₃C-(CH₂)₁₆-C(O)O-CH₂CH(OH)-CH₂(OCH₂CH₂)₃₈OH
H₃C-(CH₂)₁₆-C(O)O-CH₂CH(OH)-CH₂(OCH₂CH₂)₃₉OH
H₃C-(CH₂)₁₆-C(O)O-CH₂CH(OH)-CH₂(OCH₂CH₂)₄₀OH
H₃C-(CH₂)₁₈-C(O)O-CH₂CH(OH)-CH₂(OCH₂CH₂)₂₀OH
H₃C-(CH₂)₁₈-C(O)O-CH₂CH(OH)-CH₂(OCH₂CH₂)₂₁OH
H₃C-(CH₂)₁₈-C(O)O-CH₂CH(OH)-CH₂(OCH₂CH₂)₂₂OH
H₃C-(CH₂)₁₈-C(O)O-CH₂CH(OH)-CH₂(OCH₂CH₂)₂₃OH
H₃C-(CH₂)₁₈-C(O)O-CH₂CH(OH)-CH₂(OCH₂CH₂)₂₄OH
H₃C-(CH₂)₁₈-C(O)O-CH₂CH(OH)-CH₂(OCH₂CH₂)₂₅OH
H₃C-(CH₂)₁₈-C(O)O-CH₂CH(OH)-CH₂(OCH₂CH₂)₂₆OH
H₃C-(CH₂)₁₈-C(O)O-CH₂CH(OH)-CH₂(OCH₂CH₂)₂₇OH
H₃C-(CH₂)₁₈-C(O)O-CH₂CH(OH)-CH₂(OCH₂CH₂)₂₈OH
H₃C-(CH₂)₁₈-C(O)O-CH₂CH(OH)-CH₂(OCH₂CH₂)₂₉OH
H₃C-(CH₂)₁₈-C(O)O-CH₂CH(OH)-CH₂(OCH₂CH₂)₃₀OH
H₃C-(CH₂)₁₈-C(O)O-CH₂CH(OH)-CH₂(OCH₂CH₂)₃₁OH
H₃C-(CH₂)₁₈-C(O)O-CH₂CH(OH)-CH₂(OCH₂CH₂)₃₂OH
H₃C-(CH₂)₁₈-C(O)O-CH₂CH(OH)-CH₂(OCH₂CH₂)₃₃OH
H₃C-(CH₂)₁₈-C(O)O-CH₂CH(OH)-CH₂(OCH₂CH₂)₃₄OH
H₃C-(CH₂)₁₈-C(O)O-CH₂CH(OH)-CH₂(OCH₂CH₂)₃₅OH
H₃C-(CH₂)₁₈-C(O)O-CH₂CH(OH)-CH₂(OCH₂CH₂)₃₆OH
H₃C-(CH₂)₁₈-C(O)O-CH₂CH(OH)-CH₂(OCH₂CH₂)₃₇OH
H₃C-(CH₂)₁₈-C(O)O-CH₂CH(OH)-CH₂(OCH₂CH₂)₃₈OH
H₃C-(CH₂)₁₈-C(O)O-CH₂CH(OH)-CH₂(OCH₂CH₂)₃₉OH
H₃C-(CH₂)₁₈-C(O)O-CH₂CH(OH)-CH₂(OCH₂CH₂)₄₀OH

Ganz besonders bevorzugt ist der Einsatz von PEG-30 Glyceryl Sterate: H₃C-(CH₂)₁₆-C(O)O-CH₂CH(OH)-CH₂(OCH₂CH₂)₃₀OH

Als fünften wesentlichen Inhaltsstoff enthalten die erfindungsgemäßen Mund- und Zahnpflege-und -reinigungsmittel mindestens 0,05 bis 5,0 Gew-% mindestens eines Polyalkylenglycols der Formel (II)

H-(O-CH(R)-CH₂)m-OH (II),

in der R für -H oder -CH₃ steht und M so gewählt ist, daß das Polyalkylenglycol Molmassen zwischen 200 und 2000 Dalton aufweist.

Ganz besonders bevorzugt ist der Einsatz von Polyethylenglycolen (R = H). Erfindungsgemäß besonders bevorzugte Mund- und Zahnpflege- und -reinigungsmittel sind **dadurch gekennzeichnet, daß** sie - bezogen auf ihr Gewicht - 0,75 bis 4,5 Gew.-%, vorzugsweise 1,0 bis 4,0 Gew.-%, besonders bevorzugt 1,5 bis 3,5 und insbesondere 2,0 bis 3,0 Gew.-% Polyethylenglycol(e) mit Molmassen von 200 bis 2000 Dalton, vorzugsweise von 300 bis 1800 Dalton, besonders bevorzugt von 450 bis 1700 Dalton und insbesondere von 500 bis 1500 Dalton, enthalten.

Die erfindungsgemäßen Mund- und Zahnpflege- und -reinigungsmittel enthalten üblicherweise weitere Inhaltsstoffe.

Bevorzugt ist hierbei der Einsatz von so genannten Feuchthaltemitteln, die bei Zahnpasten das Austrocknen verhindern. Bei so genannten Flüssigzahncremes mit fließfähiger Rheologie dienen diese als Matrix und werden in höheren Mengen eingesetzt.

Hier sind erfindungsgemäße Mund und Zahnpflege- und -reinigungsmittel bevorzugt, die - bezogen auf ihr Gewicht - 10 bis 60 Gew.-%, vorzugsweise 12,5 bis 50 Gew.-%, besonders bevorzugt 15 bis 45 Gew.-% und insbesondere 20 bis 40 Gew.-% mindestens eines mehrwertigen Alkohols aus der Gruppe Sorbit und/oder Glycerin und/oder 1,2-Propylenglycol.-% enthalten. Sorbit (auch als Glucit bezeichnet) ist ein Zuckeralkohol von Glucose, also ein Hexit. Sorbit ist durch Hydrierung von Glucose herstellbar, spaltet intramolekular relativ leicht ein oder zwei Moleküle Wasser ab und bildet cyclische Ether. Sorbit läßt sich durch die Formel 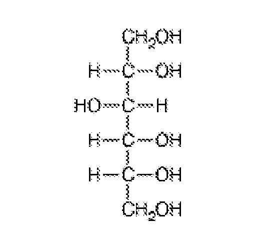 beschreiben und kommt in Form farbloser, mäßig hygroskopischer, optisch aktiver Nadeln, welche sich leicht in Wasser lösen, in den Handel.

Glycerin (1,2,3-Propantriol, 1,2,3-Trihydroxypropan, Glycerol, Ölsüß, INCI-Bezeichnung: Glycerin, E 422) ist eine farblose, klare, schwerbewegliche, geruchlose, süß schmeckende, hygroskopische Flüssigkeit, die mit Wasser und Alkohol in jedem Verhältnis mischbar ist.

### Glycerin läßt sich durch die Formel

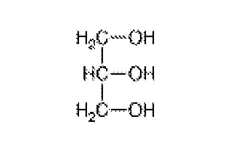

beschreiben.

1,2-Propylenglykol (1,2-Propandiol) ist eine farblose und stark hygroskopische Flüssigkeit, die in jedem Verhältnis mit Wasser und Alkoholen (wie Methanol, Ethanol, Propanolen, Butanolen) mischbar ist. Technisches 1,2-Propandiol ist ein Racemat aus (-)-(*R*)- und (+)-(*S*)-1,2-Prpylenglycol.

Für bestimmte Anwendungsbereiche kann es vorteilhaft sein, nur einen der drei oben genannten Inhaltsstoffe einzusetzen. In den meisten Fällen ist dabei Sorbit bevorzugt. Allerdings können auf anderen Anwendungsgebieten Mischungen von zwei der drei Stoffe oder aller drei Stoffe bevorzugt sein. Besonders vorteilhaft hat sich hier eine Mischung aus Glycerin, Sorbit und 1,2-Propylenglycol in einem Gewichtsverhältnis von 1 : (0,5-1) : (0,1-0,5) erwiesen.

Neben Sorbit bzw. Glycerin bzw. 1,2-Propylenglycol eignen sich als weitere mehrwertige Alkohole solche mit mindestens 2 OH-Gruppen, vorzugsweise Mannit, Xylitol und deren Mischungen. Besonders bevorzugt ist der Einsatz von Sorbit, so daß Mittel, die außer Sorbit keine anderen mehrwertigen Alkohole enthalten, besonders bevorzugt sind.

Zusätzlich zu den Fällungskieselsäuren als zwingender Inhaltsstoff können die erfindungsgemäoßen Mittel weitere Putzkörper (Abrasiva) enthalten. Putzkörper sind amorphe, überwiegend anorganische, weitgehend wasserunlösliche, kleinstteilige Pulver, die keine scharfen Kanten aufweisen. Sie begünstigen in Zahn- und Mundpflegemitteln die Reinigung der Zähne und polieren gleichzeitig die Zahnoberfläche (Poliermittel).

Als weitere Poliermittel eignen sich prinzipiell alle für Zahnpasten bekannten Reibkörper, insbesondere solche, die keine Calciumionen enthalten. Bevorzugte weitere geeignete Poliermittelkomponenten sind daher Aluminiumhydroxid, Aluminiumoxid, Natrium-aluminiumsilikate, organische Polymere oder Gemische solcher Reibkörper.

Calciumhaltige Polierkomponenten wie z.B. Kreide, Calciumpyrophosphat, Dicalcium-phosphatdihydrat können aber in Mengen bis zu 5 Gew.-% - bezogen auf die Gesamtzusammensetzung - enthalten sein.

Der Gesamtgehalt an Poliermitteln liegt vorzugsweise im Bereich von 5 - 50 Gew.-% des Zahnpflegemittels.

Als weitere Poliermittelkomponente kann auch z.B. Aluminiumoxid in Form von schwach calcinierter Tonerde mit einem Gehalt an - und -Aluminiumoxid in einer Menge von ca. 1 - 5 Gew.-% enthalten sein. Ein solches geeignetes Aluminiumoxid ist unter der Handelsbezeichnung "Poliertonerde P10 feinst" (Giulini Chemie) erhältlich.

Als Poliermittel eignen sich weiter alle für Zahnpasten bekannten Reibkörper wie z. B. Natriumaluminiumsilikate wie z. B. Zeolith A, organische Polymere wie z. B. Polymethacrylat oder Gemische dieser und der vorstehend genannten Reibkörper.

Zusammenfassend sind erfindungsgemäße Mittel bevorzugt, die zusätzlich Putzkörper, vorzugsweise Aluminiumhydroxid, Aluminiumoxid, Calciumpyrophosphat, Kreide, Dicalciumphosphat-dihydrat (CaHPO₄ ·2H₂O), Natriumaluminiumsilikate, insbesondere Zeolith A, organische Polymere, insbesondere Polymethacrylate oder Gemische dieser Reibkörper, vorzugsweise in Mengen von 1 bis 30 Gew.%, vorzugsweise von 2,5 bis 25 Gew.% und insbesondere von 5 bis 22 Gew.%, jeweils bezogen auf das gesamte Mittel, enthalten.

Mund- und Zahnpflege- und reinigungsmittel, insbesondere Zahnpasten, können z.B. auch Substanzen enthalten, die gegen Plaque und/oder Zahnstein wirksam sind.

Zahnbelag (Plaque) ist ein rauher, klebriger Belag auf den Zähnen, der aus Speichel, Bakterien und Nahrungsresten besteht. Setzen sich Mineralsalze (z.B. Calcium, Phosphat) aus dem Speichel im Zahnbelag ab, so bilden sich harte, weiße oder gelbliche Ablagerungen am Zahn, die man Zahnstein nennt. In dem porösen Zahnstein kann sich wiederum leicht Zahnbelag absetzen, der das Zahnfleisch angreift.

Die Bakterien auf der Zahnoberfläche bauen Kohlenhydrate, besonders Zucker, aus der Nahrung zu Säure ab. Diese Säure löst die Zahnsubstanz auf und es kommt zu Karies (Zahnfäule). Dabei werden besonders die Mineralien Calcium und Phosphat aus dem Zahnschmelz herausgelöst. Nach dem Zahnschmelzmantel werden auch innere Schichten des Zahnes angegriffen. Bakterien können in das Zahnmark eindringen und dort zu Entzündungen führen. Meist kommt es dann zu stechenden Zahnschmerzen.

Wie bereits erwähnt, beinhaltet Plaque Bakterien, so daß sich zur Bekämpfung von Plaque antimikrobielle Stoffe eignen. Diese besitzen darüber hinaus eine Wirkung als Konservierungsmittel.

In Mund und Zahnpflege- und -reinigungsmitteln können beispielsweise die auch in Lebensmitteln zugelassenen Konservierungsmittel Sorbinsäure (E 200), Kaliumsorbat (E 202), Calciumsorbat (E 203), Benzoesäure (E 210), Natriumbenzoat (E 211), Kaliumbenzoat (E 212), Calciumbenzoat (E 213), Ethyl-4-hydroxybenzoat (E 214), Ethyl-4-hydroxybenzoat, Natriumsalz (E 215), Propyl-4-hydroxybenzoat (E 216), Propyl-4-hydroxybenzoat, Natriumsalz (E 217), Methyl-4-hydroxybenzoat (E 218), Methyl-4-hydroxybenzoat, Natriumsalz (E 219), Schwefeldioxid (schweflige Säure), (E 220), Natriumsulfit (E 221), Natriumhydrogensulfit (E 222), Natriumdisulfit (E 223), Kaliumdisulfit (E 224), Calciumsulfit (E 226), Calciumhydrogensulfit (E 227), Kaliumhydrogensulfit (E 228), Biphenyl (E 230), Orthophenylphenol (2-Biphenylol), (E 231), Natriumorthophenylphenolat (E 232), Nisin (E 234), Natamycin (E 235), Ameisensäure (E 236), Natriumformiat (E 237), Calciumformiat (E 238), Hexamethylentetramin (E 239), Dimethyldicarbonat (E 242), Kaliumnitrit (E 249), Natriumnitrit (E 250), Natriumnitrat (E 251), Kaliumnitrat (E 252), Essigsäure (E 260), Kaliumacetat (E 261), Natriumacetat (E 262), Calciumacetat (E 263), Milchsäure (E 270), Propionsäure (E 280), Natriumpropionat (E 281), Calciumpropionat (E 282), Kaliumpropionat (E 283), Borsäure (E 284), Natriumtetraborat (E 285), Hydroxybernsteinsäure (Äpfelsäure), (E 296), Fumarsäure (E 297), Lysozym (E 1105), eingesetzt werden.

Bevorzugte Stoffe sind ausgewählt aus p-Hydroxybenzoesäuremethyl-, -ethyl- oder propylester, Natriumsorbat, Natriumbenzoat, Bromchlorophen, Triclosan, Phenyl- Salicylsäureester, Biguaniden z. B. Chlorhexidin (1,1'-Hexamethylenbis[5-(4-chlorphenyl)-biguanid), Thymol usw..

Erfindungsgemäß bevorzugte Mittel enthalten zusätzlich Antiplaque-Wirkstoffe, vorzugsweise p-Hydroxybenzoesäuremethyl-, -ethyl- oder propylester, Natriumsorbat, Natriumbenzoat, Bromchlorophen, Triclosan, Phenyl- Salicylsäureester, Biguanide z. B. Chlorhexidin, Thymol, vorzugsweise in Mengen von 0,1 bis 5 Gew.%, vorzugsweise von 0,25 bis 2,5 Gew.% und insbesondere von 0,5 bis 1,5 Gew.%, jeweils bezogen auf das gesamte Mittel.

Die angegebenen Mengen beziehen sich auf die Gesamtmenge an Antiplaque-Wirkstoffen, wobei einzelne Wirkstoffe vorzugsweise in engeren Mengenbereichen eingesetzt werden. Erfindungsgemäß bevorzugte Mittel enthalten beispielsweise 0,1 bis 2 Gew.-%, vorzugsweise 0,2 bis 1,75 Gew.-%, weiter bevorzugt 0,3 bis 1,5 Gew.-% und insbesondere 0,4 bis 1,0 Gew.-% Natriumbenzoat. Weiter bevorzugte erfindungsgemäße Mittel sind **dadurch gekennzeichnet, daß** sie 0,005 bis 0,1 Gew.-%, vorzugsweise 0,01 bis 0,075 Gew.-% und insbesondere 0,02 bis 0,05 Gew.-% Chlorhexidin enthalten. Chlorhexidin wird vorzugsweise gemeinsam mit Alkylpolyglycosiden (APG) eingesetzt, wobei in bevorzugten erfindungsgemäßen Mitteln als Alkylglykoside solche mit 8-18 C-Atomen in der Alkylgruppe und einem mittleren Oligomerisationsgrad des Glycosidrestes von 1-3 in einer Menge von 0,025 bis 2,5 Gew.-% enthalten sind.

Gegen Zahnstein wirksame Stoffe können beispielsweise Chelatbildner sein wie z. B. Ethylendiamintetraessigsäure und deren Natriumsalze, Pyrophosphat- Salze wie die wasserlöslichen Dialkali- oder Tetraalkalimetallpyrophosphat- Salze, z. B. Na₄P₂O₇, K₄P ₂O₇, Na₂K₂P₂O_{7,} Na₂H₂P₂O₇ und K ₂H₂P₂O₇ oder Polyphosphat-Salze, die z. B. aus wasserlöslichen Alkalimethalltripolyphosphaten wie Natriumtripolyphosphat und Kaliumtripolyphosphat ausgewählt sein können.

Erfindungsgemäß bevorzugte Mittel sind **dadurch gekennzeichnet, daß** sie zusätzlich Phosphat(e), vorzugsweise Alkalimetallphosphat(e) und insbesondere Natriumtripolyphosphat, vorzugsweise in Mengen von 1 bis 10 Gew.-%, besonders bevorzugt von 2 bis 8 Gew.-% und insbesondere von 3 bis 7 Gew.-%, jeweils bezogen auf das gesamte Mittel, enthalten.

Mund und Zahnpflege- und -reinigungsmittel können darüber hinaus mit besonderem Vorzug Antikaries-Wirkstoffe enthalten. Diese können beispielsweise aus organischen oder anorganischen Fluoriden ausgewählt sein, z. B. aus Natriumfluorid, Kaliumfluorid, Natriummonofluorphosphat und Natriumfluorosilikat. Auch Zinkfluorid, Zinn-(II)-fluorid sind bevorzugt. Bevorzugt sollte eine Menge von 0,01 - 0,2 Gew.-% Fluor in Form der genannten Verbindungen enthalten sein.

Erfindungsgemäße Mittel, die zusätzlich Antikaries-Wirkstoffe, vorzugsweise Fluorverbindung(en), insbesondere Natriumfluorid, Kaliumfluorid, Natriummonofluorphosphat, Zinkfluorid, Zinnfluorid und Natriumfluorosilikat, vorzugsweise in Mengen von 0,01 bis 5 Gew.%, vorzugsweise von 0,1 bis 2,5 Gew.% und insbesondere von 0,2 bis 1,1 Gew.%, jeweils bezogen auf das gesamte Mittel, enthalten, sind erfindungsgemäß bevorzugt.

Als Konsistenzregler (bzw. Bindemittel) dienen z. B. natürliche und/oder synthetische wasserlösliche Polymere wie Alginate, Carragheenate, Traganth, Stärke und Stärkeether, Celluloseether wie z. B. Carboxymethylcellulose (Na-Salz), Hydroxyethylcellulose, Methylhydroxypropylcellulose, Guar, Akaziengum, Agar-Agar, Xanthan- Gum, Succinoglycan-Gum, Johannisbrotmehl, Pectine, wasserlösliche Carboxyvinylpolymere (z. B. Carbopol®-Typen), Polyvinylalkohol, Polyvinylpyrrolidon, Polyethylenglycole, insbesondere solche mit Molekulargewichten von 1 500-1 000 000.

Weitere Stoffe, die sich zur Viskositätskontrolle eignen, sind z. B. Schichtsilikate wie z. B. Montmorillonit-Tone, kolloidale Verdickungskieselsäuren wie z. B. Aerogel-Kieselsäuren, pyrogene Kieselsäuren oder feinstvermahlene Fällungskieselsäuren. Es können auch viskositätsstabilisierende Zusätze aus der Gruppe der kationischen, zwitterionischen oder ampholytischen stickstoffhaltigen Tenside, der hydroxypropylsubstituierten Hydrocolloide oder der Polyethylenglycol/Polypropylenglycol- Copolymere mit einem mittleren Molgewicht von 1000 bis 5000 oder eine Kombination der genannten Verbindungen in den Zahnpasten verwendet werden.

Die Mund- und Zahnpflegemittel, insbesondere die Zahnpasten, können auch die Unempfindlichkeit der Zähne steigernde Substanzen enthalten, beispielsweise Kaliumsalze wie z. B. Kaliumnitrat, Kaliumcitrat, Kaliumchlorid, Kaliumbicarbonat und Kaliumoxalat. Erfindungsgemäß bevorzugte Mund- und Zahnpflege- sind **dadurch gekennzeichnet, daß** sie die Unempfindlichkeit der Zähne steigernde Substanzen, vorzugsweise Kaliumsalze, besonders bevorzugt Kaliumnitrat und/oder Kaliumcitrat und/oder Kaliumchlorid und/oder Kaliumbicarbonat und/oder Kaliumoxalat, vorzugsweise in Mengen von 0,5 bis 20 Gew.%, besonders bevorzugt von 1,0 bis 15 Gew.%, weiter bevorzugt von 2,5 bis 10 Gew.-% und insbesondere von 4,0 bis 8,0 Gew.%, jeweils bezogen auf das gesamte Mittel, enthalten.

Die erfindungsgemäßen Mittel, insbesondere die Zahnpasten, können auch zusätzlich weitere wundheilende und entzündungshemmende Stoffe, z. B. Wirkstoffe gegen Zahnfleischentzündungen, enthalten. Derartige Stoffe können z. B. ausgewählt sein aus Allantoin, Azulen, Kamillenextrakten, Tocopherol, Panthenol, Bisabolol, Salbeiextrakten.

Als nicht-kationische, bakterizide Komponente eignen sich z.B. Phenole, Resorcine, Bisphenole, Salicylanilide und deren halogenierte Derivate, halogenierte Carbanilide und p-Hydroxybenzoesäureester. Besonders bevorzugte antimikrobielle Komponenten sind halogenierte Diphenylether, z.B. 2,4-Dichlor-2'-hydroxydiphenylether, 4,4'-Dichlor-2'-hydroxydiphenylether, 2,4,4'-Tribrom-2'-hydroxydiphenylether und 2,4,4'-Trichlor-2'-hydroxydiphenylether (Triclosan). Sie werden bevorzugt in Mengen von 0,01 - 1 Gew.-% in die erfindungsgemäßen Zahnpflegemittel eingesetzt. Besonders bevorzugt wird Triclosan in einer Menge von 0,01 - 0,3 Gew.-% eingesetzt.

D-Panthenol D - (+) - 2,4-Dihydroxy-N-(3-hydroxypropyl)-3,3-dimethyl-butyramid zeigt eine der Pantothensäure entsprechende biologische Aktivität. Die Pantothensäure (R - (+) - N - (2,4-Dihydroxy-3,3-dimethylbutyryl-β-alanin) ist eine Vorstufe in der Biosynthese des Coenzyms A und wird zum Vitamin-B-Komplex (B3) gezählt. Diese Stoffe sind dafür bekannt, daß sie die Wundheilung fördern und eine günstige Wirkung auf die Haut haben. Sie sind daher auch gelegentlich in Zahnpasten beschrieben worden. Die erfindungsgemäßen Zahnpflegemittel enthalten bevorzugt 0,05 - 5 Gew.-% Panthenol oder ein Salz der Pantothensäure.

Retinol (3,7-Dimethyl-9-(2,6,6-trimethyl-1-cyclohexenyl)-2,4,6,8-nonatetraen-1-ol ist der internationale Freiname für Vitamin A1. Anstelle des Retinols kann auch eines seiner Derivate mit ähnlicher biologischer Wirkung, z.B. ein Ester oder die Retinoesäure (Tretinoin), eines ihrer Salze oder ihre Ester verwendet werden. Bevorzugt wird ein Retinol-Ester, insbesondere ein Fettsäureester einer Fettsäure mit 12 - 22 C-Atomen verwendet. Besonders bevorzugt ist Retinol-Palmitat geeignet. Bei Verwendung eines Retinol-Esters, z.B. Retinol-Palmitat mit einer Aktivität von 1,7 10⁶ I.E. pro g ist eine Menge von 0,001 bis 0,1 Gew.-% bevorzugt. Bei Verwendung anderer Retinol-Derivate empfiehlt sich eine Einsatzmenge, die einer Konzentration von 10³ bis 10⁶ I.E. (Internationale Einheiten) pro 100 g entspricht.
Bevorzugte Zahnpflegemittel gemäß der vorliegenden Erfindung enthalten neben Poliermitteln, Fluorverbindungen, Feuchthaltemitteln und Bindemitteln bevorzugt
0,01 - 1 Gew.-% eines halogenierten Diphenylethers
0,05 - 5 Gew.-% Panthenol oder ein Salz der Pantothensäure und
0,01 - 0,1 Gew.-% eines Retinol-Esters, bevorzugt Retinol-Palmitat.

Die erfindungsgemäßen Mittel, insbesondere die Zahnpasten, können auch zusätzliche Substanzen zur Erhöhung des mineralisierenden Potentials enthalten, beispielsweise calciumhaltige Substanzen wie z. B. Calciumchlorid, Calciumacetat und Dicalciumphosphat-Dihydrat. Die Konzentration der calciumhaltigen Substanz hängt von der Löslichkeit der Substanz und dem Zusammenwirken mit anderen in dem Mund- und Zahnpflegemittel enthaltenen Substanzen ab.

Neben den genannten obligatorischen Komponenten können die erfindungsgemäßen Zahnpflegemittel weitere, an sich bekannte Hilfs- und Zusatzstoffe enthalten. Dabei ist ein Zusatzstoff, der als Zahnpastenkomponente seit langem bekannt ist, in den erfindungsgemäßen Zahnpflegemitteln besonders wirksam: Calcium-glycerophosphat, das Calcium-Salz der Glycerin-1-phosphorsäure oder der Glycerin-2-phosphorsäure oder der zur Glycerin-1-phosphorsäure enantiomeren Glycerin-3-phosphorsäure - oder eines Gemisches dieser Säuren. Die Verbindung hat in Zahnpflegemitteln eine remineralisierende Wirkung, da sie sowohl Calcium- als auch Phosphationen liefert. In den erfindungsgemäßen Zahnpflegemitteln wird Calciumglycerophosphat bevorzugt in Mengen von 0,01 - 1 Gew.-% eingesetzt. Insgesamt können die erfindungsgemäßen Zahnreinigungsmittel übliche Hilfsmittel und Zusatzstoffe in Mengen bis zu 10 Gew.-% enthalten.

Die erfindungsgemäßen Zahnpflegemittel können z.B. durch Zusatz von Aromaölen und Süßungsmitteln in ihren organoleptischen Eigenschaften verbessert werden.

Als Aromaöle können alle die für Mund- und Zahnpflegemittel üblichen natürlichen und synthetischen Aromen eingesetzt werden. Natürliche Aromen können sowohl in Form der aus Drogen isolierten natürlichen ätherischen Öle als auch der daraus isolierten Einzelkomponenten enthalten sein.

Geeignete Aromen sind z.B. Pfefferminzöl, Krauseminzöl, Eukalyptusöl, Anisöl, Fenchelöl, Kümmelöl, Menthylacetat, Zimtaldehyd, Anethol, Vanillin, Thymol sowie Mischungen dieser Komponenten.

Geeignete Süßungsmittel sind z.B. Saccharin-Natrium, Natrium-Cyclamat, Sucrose, Lactose, Meltose, Fructose.

Weitere übliche Hilfs- und Zusatzstoffe für Zahnpasten sind
- Oberflächenaktive Stoffe, bevorzugt anionische, zwitterionische, amphotere, nichtionische Tenside oder eine Kombination mehrerer verschiedener Tenside
- Lösungsmittel und Lösungsvermittler, z.B. niedere einwertige oder mehrwertige Alkohole oder Ether, z.B. Ethanol, 1,2-Propylenglycol, Diethylenglycol oder Butyldi- glycol
- Pigmente, wie z.B. Titandioxid
- Farbstoffe
- Puffersubstanzen, z.B. primäre, sekundäre oder tertiäre Alkaliphosphate oder Citronensäure-/Na-Citrat
- weitere wundheilende oder entzündungshemmende Stoffe, z.B. Allantoin, Harnstoff, Azulen, Kamillewirkstoffe, Acetylsalicylsäurederivate oder Rhodanid
- weitere Vitamine wie z.B. Ascorbinsäure, Biotin, Tocopherol oder Rutin
- Mineralsalze wie z.B. Mangan-, Zink- oder Magnesiumsalze.

Eine weitere wichtige Gruppe von Inhaltstoffen, die in den erfindungsgemäßen Mitteln enthalten sein kann, sind die so genannten bioaktiven Gläser.

Der Begriff "bioaktive Gläser" umfaßt im Rahmen der vorliegenden Anmeldung Gläser, welche biologisch wirksam und/oder biologisch aktiv sind. Die biologische Wirksamkeit eines Glases kann sich beispielsweise in dessen antimikrobiellen Eigenschaften zeigen, biologisch aktives Glas unterscheidet sich von herkömmlichen Kalk-Natrium-Silicat-Gläsern dadurch, daß es lebendes Gewebe bindet. Biologisch aktives Glas bezeichnet dabei beispielsweise ein Glas, das eine feste Bindung mit Körpergewebe eingeht, wobei eine Hydroxyl-Apatitschicht ausgebildet wird. Unter bioaktivem Glas wird auch ein Glas verstanden, das antimikrobielle und/oder entzündungshemmende Wirkung zeigt. Die Glaspulver zeigen gegenüber Bakterien, Pilzen sowie Viren eine biozide bzw. eine biostatische Wirkung; sind im Kontakt mit dem Menschen hautverträglich, toxikologisch unbedenklich und insbesondere auch zum Verzehr geeignet.

Erfindungsgemäß besonders bevorzugte Mittel sind **dadurch gekennzeichnet, daß** sie 0,2 bis 20 Gew.-%, vorzugsweise 0,4 bis 14 Gew.-%, besonders bevorzugt 0,5 bis 3 Gew.-% und insbesondere 0,6 bis 2 Gew.-% mindestens eines bioaktiven Glases enthalten.

Die erfindungsgemäßen Mund- und Zahnpflege- und -reinigungsmittel dieser Ausführungsform enthalten bioaktives Glas oder Glaspulver oder Glaskeramikpulver oder Kompositmaterialien, welche ein solches bioaktives Glas umfassen. Unter Glaspulvern werden im Rahmen der vorliegenden Anmeldung auch Granulate und Glaskügelchen verstanden.

Aufgrund der Anforderungen an die toxikologische Unbedenklichkeit des Glases sowie deren Eignung zum Verzehr soll das Glaspulver besonders rein sein. Die Belastung durch Schwermetalle ist vorzugsweise gering. So beträgt die Maximalkonzentration im Bereich der kosmetischen Formulierungen vorzugsweise für Pb < 20 ppm, Cd < 5 ppm, As < 5 ppm, Sb < 10 ppm, Hg < 1 ppm, Ni < 10 ppm.

Das unkeramisierte Ausgangsglas, das direkt in den bevorzugten erfindungsgemäßen Zusammensetzungen enthalten oder gegebenenfalls für die Herstellung einer erfindungsgemäß einsetzbaren Glaskeramik verwandt wird, enthält SiO₂ als Netzwerkbildner, vorzugsweise zwischen 35-80 Gew.-%. Bei niedrigeren Konzentrationen nimmt die spontane Kristallisationsneigung stark zu und die chemische Beständigkeit stark ab. Bei höheren SiO₂-Werten kann die Kristallisationsstabilität abnehmen, und die Verarbeitungstemperatur wird deutlich erhöht, so daß sich die Heißformgebungseigenschaften verschlechtern. Na₂O wird als Flußmittel beim Schmelzen des Glases eingesetzt. Bei Konzentrationen kleiner 5% wird das Schmelzverhalten negativ beeinflußt. Natrium ist Bestandteil der sich bei der Keramisierung bildenden Phasen und muß, sofern hohe kristalline Phasenanteile durch die Keramisierung eingestellt werden sollen, in entsprechend hohen Konzentrationen im Glas enthalten sein. K₂O wirkt als Flußmittel beim Schmelzen des Glases. Außerdem wird Kalium in wässrigen Systemen abgegeben. Liegen hohe Kaliumkonzentrationen im Glas vor, werden kaliumhaltige Phasen wie Kalzium-Silicaten ebenfalls ausgeschieden. Über den P₂O₅-Gehalt kann bei silikatischen Gläsern, Glaskeramiken oder Kompositen die chemische Beständigkeit des Glases und damit die Ionenabgabe in wässrigen Medien eingestellt werden. Bei Phospahtgläsern ist P₂O₅ Netzwerkbilder. Der P₂O₅-Gehalt liegt vorzugsweise zwischen 0 und 80 Gew.-%. Um die Schmelzbarkeit zu verbessern, kann das Glas bis zu 25 Gew.- % B₂O₃ enthalten. Al₂O₃ wird genutzt, um die chemische Beständigkeit des Glases einzustellen.

Zur Verstärkung der antimikrobiellen, insbesondere der antibakteriellen Eigenschaften der Glaskeramik können antimikrobiell wirkende Ionen wie z. B. Ag, Au, I, Ce, Cu, Zn in Konzentrationen kleiner 5 Gew.-% enthalten sein.

Farbgebende Ionen wie z. B. Mn, Cu, Fe, Cr, Co, V, können einzeln oder kombiniert, vorzugsweise in einer Gesamtkonzentration kleiner 1 Gew.-%, enthalten sein.

Üblicherweise wird das Glas bzw. die Glaskeramik in Pulverform eingesetzt. Die Keramisierung kann entweder mit einem Glasblock bzw. Glasribbons erfolgen oder aber mit Glaspulver. Nach der Keramisierung müssen die Glaskeramikblöcke oder Ribbons zu Pulver gemahlen werden. Wurde das Pulver keramisiert, muß gegebenenfalls auch erneut gemahlen werden, um Agglomerate, die während des Keramisierungsschrittes entständen sind, zu entfernen. Die Mahlungen können sowohl trocken als auch in wässrigen oder nicht wässrigen Mahlmedien durchgeführt werden. Üblicherweise liegen die Partikelgrößen kleiner 500 µm. Als zweckmäßig haben sich Partikelgrößen < 100 µm bzw. < 20 µm erwiesen. Besonders geeignet sind Partikelgrößen < 10 µm sowie kleiner 5 µm sowie kleiner 2 µm, siehe weiter unten.

Die in den bevorzugten erfindungsgemäßen Zusammensetzungen enthaltenen bioaktiven Gläser bzw. Glaspulver oder Glaskeramikpulver oder Komposit-Zusammensetzungen umfassen Gläser, die bevorzugt nachfolgende Komponenten umfassen: SiO₂: 35-80 Gew.-%, Na₂O: 0-35 Gew.- %, P₂O₅: 0-80 Gew.-%, MgO: 0-5 Gew.-%, Ag₂O: 0-0,5 Gew.-%, AgJ: 0-0,5 Gew.- %, NaJ: 0-5 Gew.-%, TiO₂: 0-5 Gew.-%, K₂O: 0-35 Gew.-%, ZnO: 0-10 Gew.-%, Al ₂O₃: 0-25 Gew.-% und B₂O₃: 0-25 Gew.-% .

Weiterhin können dem Grundglas gemäß obiger Zusammensetzung zur Erzielung weiterer Effekte wie beispielsweise Farbigkeit oder UV-Filterung Ionen wie Fe, Co, Cr, V, Ce, Cu, Mn, Ni, Bi, Sn, Ag, Au, J einzeln oder in Summe bis zu 10 Gew.-% zugegeben werden. Eine weiter Glaszusammensetzung kann wie folgt sein: SiO₂: 35-80 Gew.-%, Na₂O: 0-35 Gew.-%, P₂O₅: 0-80 Gew.-%, MgO: 0-5 Gew.- %, Ag₂O: 0-0,5 Gew.-%, AgJ: 0-0,5 Gew.-%, NaJ: 0-5 Gew.-%, TiO₂: 0-5 Gew.-%, K₂O: 0-35 Gew.-%, ZnO: 0-10 Gew.-%, Al₂ O₃: 0-25 Gew.-%, B₂O₃: 0- 25 Gew.-%, SnO: 0-5 Gew.-%, CeO₂: 0-3 Gew.- % und Au: 0,001-0,1 Gew.-%.

Besonders bevorzugte erfindungsgemäße Mund- und Zahnpflege- und -reinigungsmittel sind **dadurch gekennzeichnet, daß** das bioaktive Glas - bezogen auf sein Gewicht - folgende Zusammensetzung aufweist:

| | | | |
|---|---|---|---|
| SiO₂ | 35 bis 60 Gew.-%, | vorzugsweise | 40 bis 60 Gew.-%, |
| Na₂O | 0 bis 35 Gew.-%, | vorzugsweise | 5 bis 30 Gew.-%, |
| K₂O | 0 bis 35 Gew.-%, | vorzugsweise | 0 bis 20 Gew.-%, |
| P₂O₅ | 0 bis 10 Gew.-%, | vorzugsweise | 2 bis 10 Gew.-%, |
| MgO | 0 bis 10 Gew.-%, | vorzugsweise | 0 bis 5 Gew.-%, |
| CaO | 0 bis 35 Gew.-%, | vorzugsweise | 5 bis 30 Gew.-%, |
| Al₂O₃ | 0 bis 25 Gew.-%, | vorzugsweise | 0 bis 5 Gew.-%, |
| B₂O₃ | 0 bis 25 Gew.-%, | vorzugsweise | 0 bis 5 Gew.-%, |
| TiO₂ | 0 bis 10 Gew.-%, | vorzugsweise | 0,1 bis 5 Gew.-%. |

Wie bereits weiter oben erwähnt, wird das bioaktive Glas vorzugsweise in partikulärer Form eingesetzt. Hier sind besonders bevorzugte erfindungsgemäße Mund- und Zahnpflege- und - reinigungsmittel **dadurch gekennzeichnet, daß** das antimikrobielle Glas Teilchengrößen < 10 µm, vorzugsweise von 0,5 bis 4 µm, besonders bevorzugt von 1 bis 2 µm, aufweist.

Ein weiterer Gegenstand der vorliegenden Erfindung ist die nicht-therapeutische kosmetische Verwendung von erfindungsgemäßen Mund- und Zahnpflege- und -reinigungsmitteln zur Vorbeugung und/oder Bekämpfung hypersensibler Zähne.

Ein weiterer Gegenstand der vorliegenden Erfindung ist die nicht-therapeutische kosmetische Verwendung von erfindungsgemäßen Mund- und Zahnpflege- und -reinigungsmitteln zur Aufhellung der Zähne und/oder zur Vorbeugung von Verfärbungen und Wiederverfärbungen.

Ein weiterer Gegenstand der vorliegenden Erfindung ist die nicht-therapeutische kosmetische Verwendung von erfindungsgemäßen Mund- und Zahnpflege- und -reinigungsmitteln zur Vorbeugung gegen Plaqueadhäsion auf der Zahnoberfläche.

Ein weiterer Gegenstand der vorliegenden Erfindung ist die nicht-therapeutische kosmetische Verwendung von erfindungsgemäßen Mund- und Zahnpflege- und -reinigungsmitteln zur Remineralisation von Dentalläsionen.

Bezüglich bevorzugter Ausführungsformen der erfindungsgemäßen nicht-therapeutischen Verwendungen gilt mutatis mutandis das zu den erfindungsgemäßen Mitteln Gesagte.

Ein weiterer Gegenstand der vorliegenden Erfindung ist ein Verfahren zur Remineralisation von Zähnen, bei dem man ein erfindungsgemäßes Mund- und Zahnpflege- und -reinigungsmittel auf einen zur mechanischen Reinigung der Zähne geeigneten Gegenstand aufbringt und zur Reinigung der Zahnoberflächen, Zahnzwischenräume und der Mundhöhle verwendet.

Auch bezüglich bevorzugter Ausführungsformen des erfindungsgemäßen Verfahrens gilt mutatis mutandis das zu den erfindungsgemäßen Mitteln Gesagte.

Die nachfolgenden beispiele verdeutlichen den Gegenstand der Erfindung:

### Beispiele:

Es wurden folgende Zahncremes bereitgestellt (Angaben in Gew.-%):

| | **1** | **2** | **3** | **4** | **5** |
|---|---|---|---|---|---|
| Sorbitol, 70%ig | 31,0 | 30,0 | - | - | 20,0 |
| Glycerin, 86%ig | - | - | 35,0 | 41,0 | 21,0 |
| Na-CMC (Substitutionsgrad 0,75) | 1,0 | 0,75 | 1,1 | 2,0 | 1,5 |
| Fällungskieselsäure (Sident^{®}) 8) | 9,0 | 8,0 | 12,0 | 14,0 | 8,0 |
| Fällungskieselsäure (Sident^{®}) 22 S) | 7,0 | 7,0 | 4,0 | 10,0 | 8,0 |
| C₁₈-Fettalkoholsulfat, Na-Salz | 1,5 | - | 0,75 | 1,5 | - |
| Cocoamidopropylbetain | - | 1,5 | 0,75 | - | 1,5 |
| PEG-30 Glyceryl Stearat | 0,2 | 0,25 | 0,1 | 0,5 | 1,0 |
| PEG (MG = 1500) | 1,5 | 2,0 | 3,0 | 0,5 | 1,5 |
| Natriumfluorid | 0,32 | 0,32 | 0,32 | 0,32 | 0,32 |
| Dinatriumphosphat | 0,1 | 0,1 | 0,1 | 0,1 | 0,1 |
| Trinatriumphoyphat | 0,1 | 0,1 | 0,1 | 0,1 | 0,1 |
| Saccharin, Na-Salz | 0,2 | 0,2 | 0,2 | 0,2 | 0,2 |
| Natriumbenzoat | 0,25 | 0,5 | 0,5 | 0,25 | 0,25 |
| Aroma | 1,0 | 1,0 | 1,0 | 1,0 | 1,0 |
| Wasser, vollentsalzt | ad 100 | ad 100 | ad 100 | ad 100 | ad 100 |

## Patentansprüche

1. Mund- und Zahnpflege- und -reinigungsmittel, enthaltend - bezogen auf sein Gewicht -
a) 0,1 bis 5,0 Gew.-% Na-Carboxymethylcellulose mit einem Substitutionsgrad von 0,65 bis 0,85;
b) 2,0 bis 25,0 Gew.-% Fällungskieselsäure(n);
c) 0,1 bis 2 Gew.-% mindestens eines Tensids;
d) 0,05 bis 5,0 Gew.-% mindestens eines Emulgators der Formel (I)
R-C(O)O-CH₂CH(OH)-CH₂(OCH₂CH₂)ₙOH (I),
in der
- R für einen langkettigen Alkyl- oder Alkenylrest mit 7 bis 21 Kohlenstoffatomen und
- n für Werte von 10 bis 100 steht,
e) 0,5 bis 5,0 Gew.-% mindestens eines Polyalkylenglycols der Formel (II)
H-(O-CH(R)-CH₂)m-OH (II),
in der
- R für-H oder-CH₃ steht,
- M so gewählt ist, daß das Polyalkylenglycol Molmassen zwischen 200 und 2000 Dalton aufweist.

2. Mund- und Zahnpflege- und -reinigungsmittel nach Anspruch 1, **dadurch gekennzeichnet, daß** es - bezogen auf sein Gewicht - 0,25 bis 4,0 Gew.-%, vorzugsweise 0,5 bis 3,0 Gew.-%, besonders bevorzugt 0,75 bis 2,5 und insbesondere 1,0 bis 1,75 Gew.-% Na-Carboxymethylcellulose mit einem Substitutionsgrad von 0,75 bis 0,85 enthält.

3. Mund- und Zahnpflege- und -reinigungsmittel nach einem der Ansprüche 1 oder 2, **dadurch gekennzeichnet, daß** es - bezogen auf sein Gewicht - 2,5 bis 22,5, vorzugsweise 3,5 bis 20, besonders bevorzugt 5 bis 17,5 und insbesondere 6,0 bis 15,0 Gew.-% Fällungskieselsäure(n) enthält.

4. Mund- und Zahnpflege- und -reinigungsmittel nach einem der Ansprüche 1 bis 3, **dadurch gekennzeichnet, daß** es - bezogen auf sein Gewicht - 0,25 bis 1,9 Gew.-%, vorzugsweise 0,5 bis 1,8 Gew.-%, besonders bevorzugt 0,75 bis 1,7 und insbesondere 1,0 bis 1,5 Gew.-% mindestens eines Betains der Formel (III) 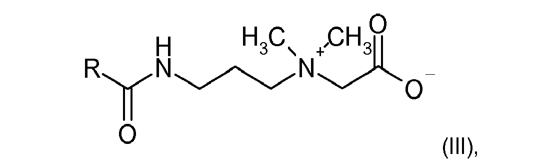 enthält, in der R für einen geradkettigen oder verzweigten, gesättigten oder ein- bzw. mehrfach ungesättigten Alkyl- oder Alkenlyrest mit 8 bis 24 Kohlenstoffatomen steht.

5. Mund- und Zahnpflege- und -reinigungsmittel nach einem der Ansprüche 1 bis 4, **dadurch gekennzeichnet, daß** es - bezogen auf sein Gewicht - 0,25 bis 1,9 Gew.-%, vorzugsweise 0,5 bis 1,8 Gew.-%, besonders bevorzugt 0,75 bis 1,7 und insbesondere 1,0 bis 1,5 Gew.-% mindestens eines Betains der Formel (IV) 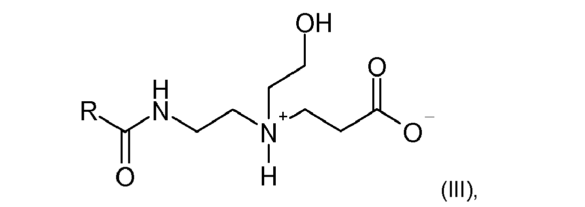 enthält, in der R für einen geradkettigen oder verzweigten, gesättigten oder ein- bzw. mehrfach ungesättigten Alkyl- oder Alkenlyrest mit 8 bis 24 Kohlenstoffatomen steht.

6. Mund- und Zahnpflege- und -reinigungsmittel nach einem der Ansprüche 4 oder 5, **dadurch gekennzeichnet, daß** der Rest R in den Formeln (III) und (IV) ausgewählt ist aus
- H₃C-(CH₂)₇-
- H₃C-(CH₂)₉-
- H₃C-(CH₂)₁₁-
- H₃C-(CH₂)₁₃-
- H₃C-(CH₂)₁₅-
- H₃C-(CH₂)₇-CH=CH-(CH₂)₇-
oder Mischungen aus diesen.

7. Mund- und Zahnpflege- und -reinigungsmittel nach einem der Ansprüche 1 bis 6, **dadurch gekennzeichnet, daß** es - bezogen auf sein Gewicht - 0,15 bis 1,9 Gew.-%, vorzugsweise 0,5 bis 1,8 Gew.-%, besonders bevorzugt 0,75 bis 1,7 und insbesondere 1,0 bis 1,5 Gew.-% mindestens eines anionischen Tensids aus der Gruppe der Fettalkoholsulfate enthält.

8. Mund- und Zahnpflege- und -reinigungsmittel nach einem der Ansprüche 1 bis 7, **dadurch gekennzeichnet, daß** es - bezogen auf sein Gewicht - 0,07 bis 4,0 Gew.-%, vorzugsweise 0,1 bis 3,0 Gew.-%, besonders bevorzugt 0,15 bis 2,0 und insbesondere 0,2 bis 1,0 Gew.-% mindestens eines eines Emulgators der Formel (I)
R-C(O)O-CH₂CH(OH)-CH₂(OCH₂CH₂)ₙOH (I),
n der
- R für -(CH2)ₖCH3 mit k 8, 10, 12, 14, 16 oder 18 und
- n für die Werte 20, 21, 22, 23, 24, 25, 26, 27, 28, 29, 30, 31, 32, 33, 34, 35, 36, 37, 38, 39 oder 40 steht.

9. Mund- und Zahnpflege- und -reinigungsmittel nach einem der Ansprüche 1 bis 8, **dadurch gekennzeichnet, daß** es - bezogen auf sein Gewicht - 0,75 bis 4,5 Gew.-%, vorzugsweise 1,0 bis 4,0 Gew.-%, besonders bevorzugt 1,5 bis 3,5 und insbesondere 2,0 bis 3,0 Gew.-% Polyethylenglycol(e) mit Molmassen von 200 bis 2000 Dalton, vorzugsweise von 300 bis 1800 Dalton, besonders bevorzugt von 450 bis 1700 Dalton und insbesondere von 500 bis 1500 Dalton, enthält.

10. Mund- und Zahnpflege- und -reinigungsmittel nach einem der Ansprüche 1 bis 9, **dadurch gekennzeichnet, daß** es die Unempfindlichkeit der Zähne steigernde Substanzen, vorzugsweise Kaliumsalze, besonders bevorzugt Kaliumnitrat und/oder Kaliumcitrat und/oder Kaliumchlorid und/oder Kaliumbicarbonat und/oder Kaliumoxalat, vorzugsweise in Mengen von 0,5 bis 20 Gew.%, besonders bevorzugt von 1,0 bis 15 Gew.%, weiter bevorzugt von 2,5 bis 10 Gew.-% und insbesondere von 4,0 bis 8,0 Gew.%, jeweils bezogen auf das gesamte Mittel, enthält.

11. Nicht-therapeutische kosmetische Verwendung von Mund- und Zahnpflege- und - reinigungsmitteln nach einem der Ansprüche 1 bis 10 zur Vorbeugung und/oder Bekämpfung hypersensibler Zähne.

12. Nicht-therapeutische kosmetische Verwendung von Mund- und Zahnpflege- und - reinigungsmitteln nach einem der Ansprüche 1 bis 10 zur Aufhellung der Zähne und/oder zur Vorbeugung von Verfärbungen und Wiederverfärbungen.

13. Nicht-therapeutische kosmetische Verwendung von Mund- und Zahnpflege- und - reinigungsmitteln nach einem der Ansprüche 1 bis 10 zur Vorbeugung gegen Plaqueadhäsion auf der Zahnoberfläche.

14. Nicht-therapeutische kosmetische Verwendung von Mund- und Zahnpflege- und - reinigungsmitteln nach einem der Ansprüche 1 bis 10 zur Remineralisation von Dentalläsionen.

15. Verfahren zur Remineralisation von Zähnen, **dadurch gekennzeichnet, daß** man ein Mund- und Zahnpflege- und -reinigungsmittel nach einem der Ansprüche 1 bis 10 auf einen zur mechanischen Reinigung der Zähne geeigneten Gegenstand aufbringt und zur Reinigung der Zahnoberflächen, Zahnzwischenräume und der Mundhöhle verwendet.
